# EUROPEAN PATENT APPLICATION

(11) **EP 1 253 156 A2**
(43) Date of publication of application: **30.10.2002**
(21) Application number: 02016469.5
(22) Date of filing: 28.07.1995
(51) Int. Cl.: C08F 4/80

(54) **High activity ruthenium or osmium metal carbene complexes for olefin metathesis reactions and synthesis and application thereof**

(30) Priority: 29.07.1994 US 282826; 29.07.1994 US 282827
(62) Divisional of application: 95929340.8
(71) Applicant: CALIFORNIA INSTITUTE OF TECHNOLOGY, Pasadena California 91125 (US)
(72) Inventor: Grubbs, Robert H., South Pasadena, California 91030 (US); Nguyen, Sonbinh T., Pasadena, California 91125 (US); Johnson, Lynda K., Carrboro, North Carolina 27510 (US); Hillmyer, Marc A., Dept. of Chem.Eng. and Material, 421 Washington Ave., SE MN 55455-0132 (US); Fu, Gregory C., Cambridge, Massachusetts 02142 (US)
(74) Representative: Daniels, Jeffrey Nicholas

(57) **Abstract**

A method for olefin metathesis polymerization, comprising contacting an olefin with a compound of the formula 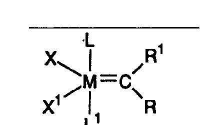 wherein:
the olefin is dicyclopentadiene;
M is Os or Ru;
R and R¹ are independently selected from hydrogen or a hydrocarbon selected from the group consisting of C₂-C₂₀ alkenyl, C₂-C₂₀ alkynyl, C₁-C₂₀ alkyl, aryl, C₁-C₂₀ carboxylate, C₂-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, and C₁-C₂₀ alkylsulfinyl;
X and X¹ are independently selected from any anionic ligand; and
L is any neutral electron donor; and
L¹ is a trialkylphosphine ligand where at least one of the alkyl groups is a secondary alkyl or a cycloalkyl.

## Description

### ORIGIN OF INVENTION

The U.S. Government has certain rights in this invention pursuant to Grant No. CHE-8922072 awarded by the National Science Foundation.

### BACKGROUND OF THE INVENTION

This invention relates to highly active and stable ruthenium or osmium metal carbene complex compounds, synthesis methods thereof and their use as catalysts in olefin metathesis reactions.

During the past two decades, research efforts have enabled an in-depth understanding of the olefin metathesis reaction as catalyzed by early transition metal complexes. In contrast, the nature of the intermediates and the reaction mechanism for Group VIII transition metal catalysts has remained elusive. In particular, the oxidation states and ligation of the ruthenium and osmium metathesis intermediates are not known.

Many ruthenium and osmium metal carbenes have been reported in the literature (for example, see Burrell, A.K., Clark, G.R., Rickard, C.E.F., Roper, W.R., Wright, A.H., J. Chem. Soc., Dalton Trans., 1991, Issue 1, pp. 609-614). However, the discrete ruthenium and osmium carbene complexes isolated to date do not exhibit metathesis activity to unstrained olefins. (Ivin, Olefin Metathesis pp. 34-36, Academic Press: London, 1983).

### SUMMARY OF THE INVENTION

The present invention relates to ruthenium or osmium carbene compounds which are stable in the presence of a variety of functional groups and which can be used to catalyze olefin metathesis reactions on unstrained cyclic and acyclic olefins.

Specifically, the present invention relates to carbene compounds of the formula 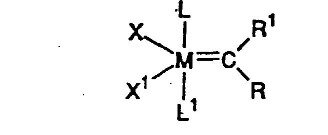 wherein:
M is Os or Ru;
R and R¹ are independently selected from hydrogen or a hydrocarbon selected from the group consisting of C₂-C₂₀ alkenyl, C₂-C₂₀ alkynyl, C₁-C₂₀ alkyl, aryl, C₁-C₂₀ carboxylate, C₂-C₂₀ alkoxy, C₂-C₂₀ alkenyloxy, C₂-C₂₀ alkynyloxy, aryloxy, C₂-C₂₀ alkoxycarbonyl, C₁-C₂₀ - alkylthio, C₁-C₂₀ alkylsulfonyl and C₁-C₂₀ alkylsulfinyl;
X and X¹ are independently selected from any anionic ligand; and
L and L¹ are independently selected from any neutral electron donor, preferably phosphine, sulfonated phosphine, phosphite, phosphinite, phosphonite, arsine, stibine, ether, amine, amide, sulfoxide, carboxyl, nitrosyl, pyridine and thioether, most preferably trialkylphosphine ligands where at least one of the alkyl groups is a secondary alkyl or a cycloalkyl.

In a preferred embodiment, the hydrocarbon is selected from the group consisting of C₁-C₅ alkyl, halogen, C₁-C₅ alkoxy and a phenyl group. The hydrocarbon also may be substituted with a C₁-C₅ alkyl halogen, C₁-C₅ alkoxy, or a phenyl group.

In an alternative embodiment, the phenyl group is optionally substituted with halogen, C₁-C₅ alkyl or C₁-C₅ alkoxy.

In a preferred embodiment, all of the alkyl groups of the trialkyl phosphine are either a secondary alkyl or a cycloalkyl. In a more preferred embodiment, the alkyl groups are either isopropyl, isobutyl, sec-butyl, neopentyl, neophenyl, cyclopentyl or cyclohexyl.

Carbene compounds where L and L¹ ligands are alkyl phosphines where the carbon backbone of at least one alkyl group of the alkyl phosphine is a secondary alkyl or cycloalkyl have been found to possess higher metathesis activity, enabling these compounds to coordinate to and catalyze metathesis reactions between all types of olefins. By contrast, previous metathesis catalysts were only able to catalyze metathesis reactions involving highly strained olefins. As a result, a broad array of metathesis reactions are enabled using the carbene compounds of the present invention that cannot be performed using less reactive catalysts.

The present invention also relates to the synthesis of ruthenium or osmium carbene compounds which can be used to catalyze olefin metathesis reactions.

Certain of the carbene compounds of the present invention are the only Ru and Os carbene complexes known to date in which the metal is formally in the +2 oxidation state, have an electron count of 16, and are pentacoordinate. Unlike most metathesis catalysts presently known which are poisoned by functional groups, the carbene compounds of the present invention are stable in the presence of alcohol, thiol, ketone, aldehyde, ester, ether, amine, amide, nitro acid, carboxylic acid, disulfide, carbonate, carboalkoxy and halogen functional groups and may therefore be used in protic or aqueous solvent systems.

In another embodiment of the present invention, the carbene compounds can be in the form wherein 2, 3 or 4 of the moieties X, X¹, L, and L¹ can be taken together to form a chelating multidentate ligand. In one aspect of this embodiment, X, L, and L¹ can be taken together to form a cyclopentadienyl, indenyl, or fluorenyl moiety.

The ruthenium or osmium carbene compounds may be prepared by reacting a compound of the formula (XX¹MLₙL¹ₘ)ₚ, in the presence of solvent, with a cyclopropene of the formula 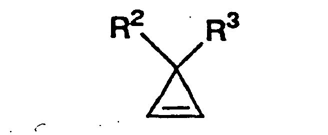 wherein:
M, X, X¹, L, and L¹ have the same meaning as indicated above;
n and m are independently 0-4, provided n+m= 2, 3 or 4;
p is an integer equal to or greater than 1; and
R² and R³ are independently selected from hydrogen or a hydrocarbon selected from the group consisting of C₂-C₁₈ alkyl, C₂-C₁₈ alkenyl, C₂-C₁₈ alkynyl, C₂-C₁₈ alkoxycarbonyl, aryl, C₁-C₁₈ carboxylate, C₁-C₁₈ alkenyloxy, C₂-C₁₈ alkynyloxy, C₁-C₁₈ alkoxy, aryloxy, C₁-C₁₈ alkylthio, C₁-C₁₈ alkylsulfonyl or C₁-C₁₈ alkylsulfinyl;

In a preferred embodiment the hydrocarbon is substituted with C₁-C₅ alkyl, halogen, C₁-C₅ alkoxy or with a phenyl group.

In a preferred embodiment the phenyl group is substituted with halogen, C₁-C₅ alkyl or C₁-C₅ alkoxy.

In one embodiment of the process, X, L, and L¹ are taken together to form a moiety selected from the group consisting of cyclopentadienyl, indenyl or fluorenyl, each optionally substituted with hydrogen; C₂-C₂₀ alkenyl, C₂-C₂₀ alkynyl, C₁-C₂₀ alkyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl; each optionally substituted with C₁-C₅ alkyl, halogen, C₁-C₅ alkoxy or with a phenyl group optionally substituted with halogen, C₁-C₅ alkyl or C₁-C₅ alkoxy.

A further method of preparing the compounds of this invention comprises reacting compound of the formula (XX¹MLₙL¹ₘ)ₚ in the presence of solvent with a phosphorane of the formula 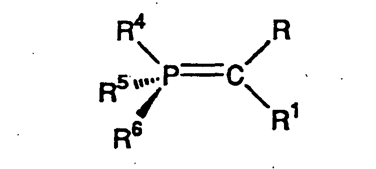 wherein:
M, X, X¹, L, L¹, n, m, p, R, and R¹ have the same meaning as indicated above; and
R⁴, R⁵ and R⁶ are independently selected from aryl, C₁-C₆ alkyl, C₁-C₆ alkoxy or phenoxy, each optionally substituted with halogen, C₁-C₃ alkyl, C₁-C₃ alkoxy, or with a phenyl group optionally substituted with halogen, C₁-C₅ alkyl or C₁-C₅ alkoxy.

The present invention also pertains to a preferred method of preparing the aforementioned ruthenium and osmium compounds comprising reacting [(Ar) MX X¹]₂ dimer complex with two equivalents of a phosphine ligand and a cyclopropene of the formula 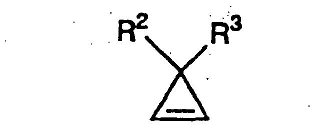 in a one step synthesis wherein:
M, X and X¹ have the same meaning as indicated above;
Ar is an aromatic compound, preferably a di-, tri-, tetra- or hexa- substituted benzene, most preferably selected from benzene, toluene, xylene, cymene, tetramethylbenzene or hexamethylbenzene; and phosphine ligand is represented by the formula PR⁷R⁸R⁹ wherein R⁷, R⁸ and R⁹ are independently selected from substituted and unsubstituted C₁-C₁₀ alkyl, secondary alkyl, cycloalkyl and aryl.

Another embodiment of the present invention comprises preparing compounds of Formula II 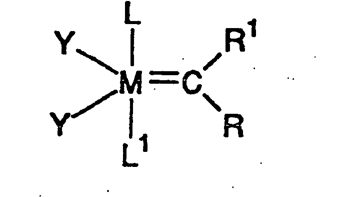 and Formula III 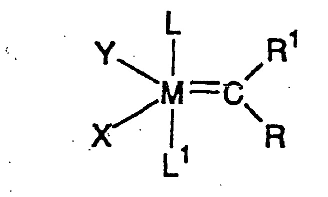 from compound of Formula I 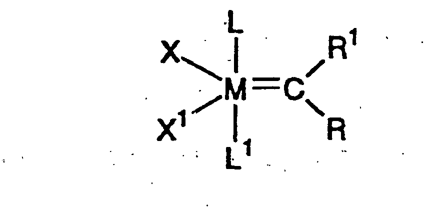 comprising reacting said compound of Formula I, in the presence of solvent, with compound of the formula M¹Y wherein:
M, R, R¹ X, X¹, L, and L¹ have the same meaning as indicated above, and wherein:
   (1) M¹ is Li, Na or K, and Y is C₁-C₁₀ alkoxide, arylalkoxide, amide or arylamide each optionally substituted with C₁-C₁₀ alkyl or halogen, diaryloxide; or
   (2) M¹ is Na or Ag, and Y is ClO₄, PF₆, BF₄, SbF₆, halogen, B(aryl)₄, C₁-C₁₀ alkyl sulfonate or aryl sulfonate.

Another embodiment of the present invention is a method of preparing compounds of structures of Formula IV 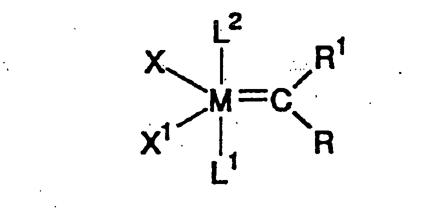 and Formula V 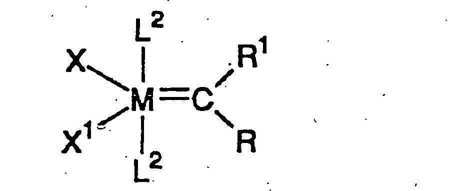 from a compound of Formula I 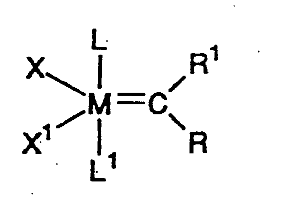 comprising reacting a compound of
Formula I, in the presence of
solvent, with L² wherein:
M, R, R¹, X, and X¹ have the same meaning as indicated above; and
L, L¹, and L² are independently selected from any neutral electron donor, preferably secondary alkyl or cycloalkyl phosphine ligands.

The compounds of Formulae II, III, IV, and V are species of, i.e., fall within, the scope of compounds of Formula I. In other words, certain compounds of Formula I are used to form other compounds of Formula I by ligand exchange. In this case, X and X¹ in Formula I are other than the Y in Formulae II and III that replaces X. Similarly, L and L¹ in Formula I are other than the L² in Formulae IV and V. If any 2 or 3 of X, X¹, L, and L¹ form a multidentate ligand of Formula I, only the remaining ligand moieties would be available for ligand replacement.

The reference above to X, X¹, L, and L¹ having the same meaning as indicated above refers to these moieties individually and taken together to form a multidentate ligand as described above.

The present invention also relates to metathesis coupling of olefins catalyzed by the carbene compounds of the present invention. The high level metathesis activity of the ruthenium and osmium carbene compounds of the present invention enable these compounds to coordinate with and catalyze metathesis reactions between all types of olefins. By contrast, previous non-carbene ruthenium and osmium metathesis catalysts are only able to catalyze metathesis reactions involving highly strained olefins. As a result, a broad array of metathesis reactions are enabled using the carbene compounds of the present invention that cannot be performed using less reactive catalysts.

Examples of metathesis olefin coupling reactions enabled by the ruthenium and osmium carbene compounds of the present invention include, but are not limited to, ring-opening metathesis polymerization of strained and unstrained cyclic olefins, ring closing metathesis of acyclic dienes, cross metathesis reactions involving at least one acyclic or unstrained cyclic olefin and depolymerization of olefinic polymers.

### DETAILED DESCRIPTION

The present invention relates to new highly active and stable ruthenium or osmium carbene compounds which can be used to catalyze olefin metathesis reactions.

Specifically, the present invention relates to carbene compounds of the formula 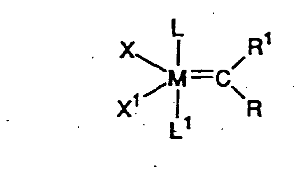 wherein:
M is Os or Ru;
R and R¹ are independently selected from hydrogen; C₂-C₂₀ alkenyl, C₂-C₂₀ alkynyl, C₁-C₂₀ alkyl, aryl, C₁-C₂₀ carboxylate, C₂-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl or C₁-C₂₀ alkylsulfinyl; each
optionally, substituted with C₁-C₅ alkyl, halogen, C₁-C₅ alkoxy or with a phenyl group optionally substituted with halogen, C₁-C₅ alkyl or C₁-C₅ alkoxy;
X and X¹ are independently selected from any anionic ligand; and
L and L¹ are independently selected from any neutral electron donor, preferably phosphine, sulfonated phosphine, phosphite, phosphinite, phosphonite, arsine, stibine, ether, amine, amide, sulfoxide, carboxyl, nitrosyl, pyridine and thioether, most preferably trialkylphosphine ligands where at least one of the alkyl groups is a secondary alkyl or a cycloalkyl.

In a preferred embodiment, all of the alkyl groups of the trialkyl phosphine are either a secondary alkyl or a cycloalkyl. In a more preferred embodiment, the alkyl groups are either isopropyl, isobutyl, sec-butyl, neopentyl, neophenyl, cyclopentyl or cyclohexyl.

The high level metathesis activity of the carbene compounds of the present invention is observed when L and L¹ are alkyl phosphines where the carbon backbone of at least one alkyl group of the alkyl phosphine is a secondary alkyl or cycloalkyl. Substitution of the secondary alkyl and cycloalkyl with additional carbon moieties and/or other functional groups are intended to be included with the terms secondary alkyl and cycloalkyl.

The ruthenium or osmium carbene complexes of the invention are useful for catalyzing olefin metathesis reactions. The propagating carbene moiety has been found to be stable and continues to polymerize additional aliquots of monomer for a period after the original amount of monomer has been consumed. The propagating carbene moiety has also been found to be stable in the presence of alcohol, thiol, ketone, aldehyde, ester, ether, amine, amide, nitro acid, carboxylic acid, disulfide, carbonate, carboalkoxy and halogen functional groups. Aspects of this invention include the metal carbene compounds, methods for their synthesis, as well as their use as catalysts in a wide variety of olefin metathesis reactions.

The intermediate compounds (XX¹MLₙL¹ₘ)ₚ are either available commercially or can be prepared by standard known methods.

The phosphorane and cyclopropene reactants used in the present invention may be prepared in accordance with the following respective references. Schmidbaur, H., et al., Phosphorus and Sulfur, Vol. 18, pp. 167-170 (1983); Carter, F.L., Frampton, V.L., Chemical Reviews, Vol. 64, No. 5 (1964), which are incorporated herein by reference.

The present invention also relates to metathesis coupling of olefins catalyzed by the carbene compounds of the present invention. The high level metathesis activity of the ruthenium or osmium carbene compounds of the present invention cause these compounds to coordinate with and catalyze metathesis reactions between all types of olefins. By contrast, previous non-carbene ruthenium or osmium metathesis catalysts are only able to catalyze metathesis reactions involving strained olefins. As a result, a broad array of metathesis reactions are enabled using the carbene compounds of the present invention that cannot be performed using less reactive catalysts.

Examples of reactions enabled by the ruthenium and osmium carbene compounds of the present invention include, but are not limited to, ring-opening metathesis polymerization of strained and unstrained cyclic olefins, ring closing metathesis of acyclic dienes, cross metathesis reactions involving at least one acyclic or unstrained cyclic olefin and depolymerization of olefinic polymers.

The carbene compounds disclosed in the present invention, as well as those disclosed in U.S. Serial No. 863,606, filed April 3, 1992, are the only Ru and Os carbene complexes known to date in which the metal is formally in the +2 oxidation state (the carbene fragment is considered to be neutral), have an electron count of 16, and are pentacoordinate. Unlike most metathesis catalysts presently known which are poisoned by functional groups, the carbene compounds of the present invention are stable in the presence of a wide variety of functional groups including alcohol, thiol, ketone, aldehyde, ester, ether, amine, amide, nitro acid, carboxylic acid, disulfide, carbonate, carboalkoxy acid, carboxylic acid, disulfide, carbonate, isocyanate, carbodiimide carboalkoxy and halogen functional groups. As a result of their stability in the presence of functional groups, these catalysts may be employed in protic and aqueous solvents as well as mixtures of protic, aqueous, and/or organic solvents.

With regard to compounds of Formula I:
alkenyl can include vinyl, 1-propenyl, 2-propenyl; 3-propenyl and the different butenyl, pentenyl and hexenyl isomers, 1,3-hexadienyl and 2,4,6-heptatrienyl, and cycloalkenyl;
alkenyloxy can include H₂C=CHCH₂O, (CH₃)₂C=CHCH₂O, (CH₃)CH=CHCH₂O, CH₃CH=CHCH₂O, (CH₃)CH=C(CH₃)CH₂O and CH₂=CHCH₂CH₂O;
alkoxide can include methoxide, *t*-butoxide and phenoxide;
alkoxy can include methoxy, ethoxy, n-propyloxy, isopropyloxy and the different butoxy, pentoxy and hexyloxy isomers;
cycloalkoxy can include cyclopentyloxy and cyclohexyloxy;
alkoxyalkyl can include CH₃OCH₂, CH₃OCH₂CH₂, CH₃CH₂OCH₂, CH₃CH₂CH₂CH₂OCH₂ and CH₃CH₂OCH₂CH₂; and
alkoxycarbonyl can include CH₃OC(=O); CH₃CH₂OC(=O), CH₃CH₂CH₂OC(=O), (CH₃)₂CHOC(=O) and the different butoxy-, pentoxy- or hexyloxycarbonyl isomers;
alkyl can include methyl, ethyl, *n*-propyl, *i*-propyl, or the several butyl, pentyl or hexyl isomers and primary, secondary and cycloalkyl isomers;
alkylsulfinyl can include CH₃SO, CH₃CH₂SO, CH₃CH₂CH₂SO, (CH₃)₂CHSO and the different butylsulfinyl, pentylsulfinyl and hexylsulfinyl isomers;
alkylsulfonyl can include CH₃SO₂, CH₃CH₂SO₂, CH₃CH₂CH₂SO₂, (CH₃)₂CHSO₂ and the different butylsulfonyl, pentylsulfonyl and hexylsulfonyl isomers;
alkylthio can include, methylthio, ethylthio, and the several propylthio, butylthio, pentylthio and hexylthio isomers;
alkynyl can include ethynyl, 1-propynyl, 3-propynyl and the several butynyl, pentynyl and hexynyl isomers, 2,7-octadiynyl and 2,5,8-decatriynyl;
alkynyloxy can include HC=CCH₂O, CH₃C=CCH₂O and CH₃C=CCH₂OCH₂O;
amide can include HC(=O)N(CH₃)₂ and (CH₃)C(=O)N(CH₃)₂;
amine can include tricyclohexylamine, triisopropylamine and trineopentylamine;
arsine can include triphenylarsine,tricyclohexylarsine and triisopropylarsine;
aryl can include phenyl, p-tolyl and p-fluorophenyl;
carboxylate can include CH₃CO₂CH₃CH₂CO₂, C₆H₅CO₂, (C₆H₅)CH₂CO₂;
cycloalkenyl can include cyclopentenyl and cyclohexenyl.
cycloalkyl can include cyclopropyl, cyclobutyl, cyclopentyl and cyclohexyl;
diketonates can include acetylacetonate and 2,4-hexanedionate;
ether can include (CH₃)₃CCH₂OCH₂CH₃, THF, (CH₃)₃COC(CH₃)₃, CH₃OCH₂CH₂OCH₃, and CH₃OC₆H₅;
phosphine can include triphenylphosphine, tricyclohexylphosphine, triisopropylphosphine, trineopentylphosphine and methyldiphenylphosphine;
phosphinite can include triphenylphosphinite, tricyclohexylphosphinite, triisopropylphosphinite, and methyldiphenylphosphinite;
phosphite can include triphenylphosphite, tricyclohexylphosphite, tri-t-butylphosphite, triisopropylphosphite and methyldiphenylphosphite;
secondary alkyl includes ligands of the general formula -CHRR¹ where R and R¹ are carbon moieties;
stibine can include triphenylstibine, tricyclohexylstibine and trimethylstibine;
sulfonate can include trifluoromethanesulfonate, tosylate, and mesylate;
sulfoxide can include CH₃S(=O)CH₃, (C₆H₅)₂SO; and
thioether can include CH₃SCH₃, C₆H₅SCH₃, CH₃OCH₂CH₂SCH₃, and tetrahydrothiophene.

A neutral electron donor is any ligand which, when removed from a metal center in its closed shell electron configuration, has a neutral charge, i.e., is a Lewis base.

"halogen" or "halide", either alone or in compound words such as "haloalkyl", denotes fluorine, chlorine, bromine or iodine.

An anionic ligand is any ligand which when removed from a metal center in its closed shell electron configuration has a negative charge. An important feature of the carbene compounds of this invention is the presence of the ruthenium or osmium in the formal +2 oxidation state (the carbene fragment is considered to be neutral), an electron count of 16 and pentacoordination. A wide variety of ligand moieties X, X¹, L, and L¹ can be present and the carbene compound will still exhibit its catalytic activity.

A preferred embodiment of the carbene compounds of the present invention is a compound of the invention of Formula I wherein:
R and R¹ are independently selected from hydrogen, vinyl, C₁-C₁₀ alkyl, aryl, C₁-C₁₀ carboxylate, C₂-C₁₀ alkoxycarbonyl, C₁-C₁₀ alkoxy, aryloxy, each optionally substituted with C₁-C₅ alkyl, halogen, C₁-C₅ alkoxy or with a phenyl group optionally substituted with halogen, C₁-C₅ alkyl or C₁-C₅ alkoxy; and
X and X¹ are independently selected from halogen, hydrogen, diketonates, or C₁-C₂₀ alkyl, aryl, C₁-C₂₀ alkoxide, aryloxide, C₂-C₂₀ alkoxycarbonyl, arylcarboxylate, C₁-C₂₀ carboxylate, aryl or C₁-C₂₀ alkylsulfonate, C₁-C₂₀ alkylthio, C₁-C₂₀ alkylsulfonyl, C₁-C₂₀ alkylsulfinyl, each optionally substituted with C₁-C₅ alkyl, halogen, C₁-C₅ alkoxy or with a phenyl group optionally substituted with halogen, C₁-C₅ alkyl or C₁-C₅ alkoxy; and
L and L¹ are independently selected from phosphine, sulfonated phosphine, phosphite, phosphinite, phosphonite, arsine, stibine, ether, amine, amide, sulfoxide, carbonyl, nitrosyl, pyridine or thioether.

A more preferred embodiment of the carbene compounds of the present invention is a compound of Formula I wherein:
R and R¹ are independently selected from hydrogen; vinyl, C₁-C₅ alkyl, phenyl, C₂-C₅ alkoxycarbonyl, C₁-C₅ carboxylate, C₁-C₅ alkoxy, phenoxy; each optionally substituted with C₁-C₅ alkyl, halogen, C₁-C₅ alkoxy or a phenyl group optionally substituted with halogen, C₁-C₅ alkyl or C₁-C₅ alkoxy;
X and X¹ are independently selected from Cl, Br, I, or benzoate, acetylacetonate, C₁-C₅ carboxylate, C₁-C₅ alkyl, phenoxy, C₁-C₅ alkoxy, C₁-C₅ alkylthio, aryl, and C₁-C₅ alkyl sulfonate; each optionally substituted with C₁-C₅ alkyl or a phenyl group optionally substituted with halogen, C₁-C₅ alkyl or C₁-C₅ alkoxy; and
L and L¹ are independently selected from aryl, C₁-C₅ alkyl, secondary alkyl or cycloalkylphosphine, aryl- or C₁-C₁₀ alkylsulfonated phosphine, aryl or C₁-C₁₀ alkylphosphinite, aryl- or C₁-C₁₀ alkylphosphonite, aryl- or C₁-C₁₀ alkylphosphite, aryl- or C₁-C₁₀ alkylarsine, aryl- or C₁-C₁₀ alkylamine, pyridine, aryl- or C₁-C₁₀ alkyl sulfoxide, aryl- or C₁-C₁₀ alkylether, or aryl- or C₁-C₁₀ alkylamide, each optionally substituted with a phenyl group optionally substituted with halogen, C₁-C₅ alkyl or C₁-C₅ alkoxy.

A further preferred embodiment of the present invention is carbene compounds of Formula I wherein:
R and R¹ are independently vinyl, H, Me, Ph;
X and X¹ are independently Cl, CF₃CO₂, CH₃CO₂, CFH₂CO₂, (CH₃)₃CO, (CF₃)₂(CH₃)CO, (CF₃) (CH₃)₂CO, PhO, MeO, EtO, tosylate, mesylate, or trifluoromethanesulfonate; and
L and L¹ are independently PPh₃, P(p-Tol)₃, P(o-Tol)₃, PPh(CH₃)₂, P(CF₃)₃, P(p-FC₆H₄)₃, pyridine, P(p-CF₃C₆H₄)₃, (p-F)pyridine, (p-CF₃)pyridine, P(C₆H₄-SO₃Na)₃, P(CH₂C₆H₄-SO₃Na)₃, P(ⁱPr)₃, P(CHCH₃(CH₂CH₃))₃, P(cyclopentyl)₃, P(cyclohexyl)₃, P(neopentyl)₃ and P(neophenyl)₃.

For any of the foregoing described preferred groups of compounds, any 2, 3, or 4 of X, X¹, L, L¹ can be taken together to form a chelating multidentate ligand. Examples of bidentate ligands include, but are not limited to, bisphosphines, dialkoxides, alkyldiketonates, and aryldiketonates. Specific examples include Ph₂PCH₂CH₂PPh₂, Ph₂AsCH₂CH₂AsPh₂, Ph₂PCH₂CH₂C(CF₃)₂O-, binaphtholate dianions, pinacolate dianions, Me₂P(CH₂)₂PMe₂ and OC(CH₃)₂(CH₃)₂CO. Tridentate ligands include, but are not limited to, (CH₃)₂NCH₂CH₂P(Ph)CH₂CH₂N(CH₃)₂. Other preferred tridentate ligands are those in which X, L, and L¹ are taken together to be cyclopentadienyl, indenyl or fluorenyl, each optionally substituted with C₂-C₂₀ alkenyl, C₂-C₂₀ alkynyl, C₁-C₂₀ alkyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, C₁-C₂₀ alkylsulfinyl, each optionally substituted with C₁-C₅ alkyl, halogen, C₁-C₅ alkoxy or with a phenyl group optionally substituted with halogen, C₁-C₅ alkyl or C₁-C₅ alkoxy. More preferably in compounds of this type, X, L, and L¹ are taken together to be cyclopentadienyl or indenyl, each optionally substituted with hydrogen; vinyl, C₁-C₁₀ alkyl, aryl, C₁-C₁₀ carboxylate, C₂-C₁₀ alkoxycarbonyl, C₁-C₁₀ alkoxy, aryloxy, each optionally substituted with C₁-C₅ alkyl, halogen, C₁-C₅ alkoxy or with a phenyl group optionally substituted with halogen, C₁-C₅ alkyl or C₁-C₅ alkoxy. Most preferably, X, L, and L¹ are taken together to be cyclopentadienyl, optionally substituted with vinyl, hydrogen, Me or Ph. Tetradentate ligands include, but are not limited to O₂C(CH₂)₂P(Ph)(CH₂)₂P(Ph)(CH₂)₂CO₂, phthalocyanines, and porphyrins.

Carbene compounds of Formula I wherein L and L¹ are alkyl phosphines where at least one alkyl group is either a secondary alkyl or a cycloalkyl. These carbene compounds have been found to be more stable, more reactive to nonsterically strained cyclic alkenes and unreactive to a wider variety of substituents. (Nguyen, S., et al., J. Am. Chem. Soc., 1993, 115:9858-9859; Fu, G., et al., J. Am. Chem. Soc., 1993, 115:9856-9557.)

Specifically, carbene compounds wherein L and L¹ are triisopropyl phosphine or tricyclohexyl phosphine have been found to be stable in the presence of oxygen, moisture, adventitious impurities thereby enabling reactions to be conducted in reagent grade solvents in air (Fu, G., et al., J. Am. Chem. Soc., 1993, 115:9856-9857). Further these carbenes are stable in the presence of alcohol, thiol, ketone, aldehyde, ester, ether, amine, amide, nitro acid, carboxylic acid, disulfide, carbonate, carboalkoxy and halogen functional groups. In addition, these carbene can catalyze olefin metathesis reactions on acyclic oleinfs and strained cyclic olefins.

The most preferred carbene compounds of the present invention include: 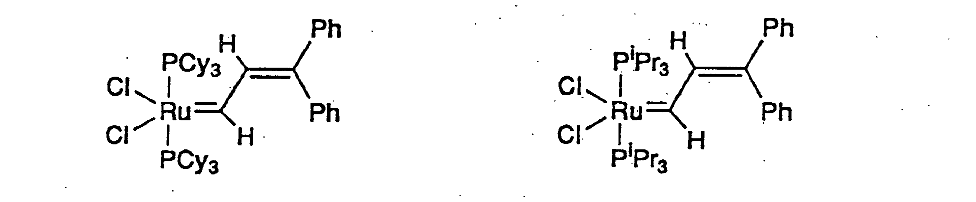 wherein
ⁱPr = isopropyl
Cy = cyclohexyl

The compounds of the present invention can be prepared in several different ways, each of which is described below.

The most general method for preparing the compounds of this invention comprises reacting (XX¹MLₙL¹ₘ)ₚ with a cyclopropene or phosphorane in the presence of a solvent to produce a carbene complex, as shown in the equations below: 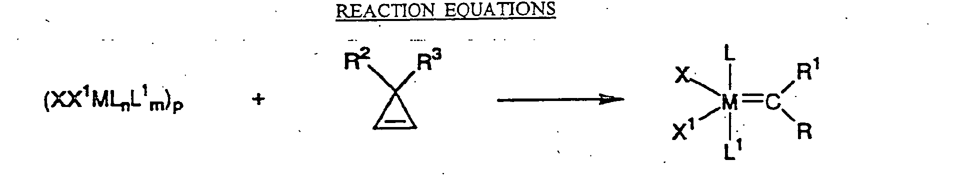 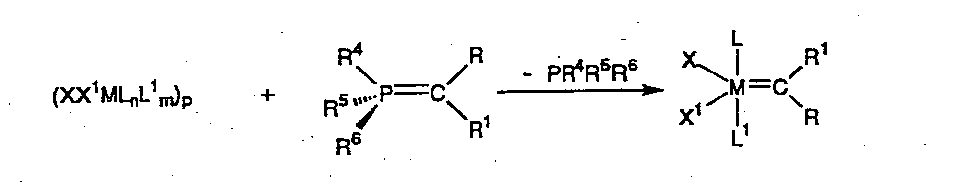 wherein:
M, X, X¹, L, L¹, n, m, p, R², R³, R⁴, R⁵, and R⁶ are as defined above. Preferably, R², R³, R⁴, R⁵, and R⁶ are independently selected from the group consisting of C₁-C₆ alkyl or phenyl.

Examples of solvents that may be used in this reaction include organic, protic, or aqueous solvents which are inert under the reaction conditions, such as: aromatic hydrocarbons, chlorinated hydrocarbons, ethers, aliphatic hydrocarbons, alcohols, water, or mixtures thereof. Preferred solvents include benzene, toluene, *p*-xylene, methylene chloride, dichloroethane, dichlorobenzene, chlorobenzene, tetrahydrofuran, diethylether, pentane, methanol, ethanol, water, or mixtures thereof. More preferably, the solvent is benzene, toluene, *p*-xylene, methylene chloride, dichloroethane, dichlorobenzene, chlorobenzene, tetrahydrofuran, diethylether, pentane, methanol, ethanol, or mixtures thereof. Most preferably, the solvent is toluene or a mixture of benzene and methylene chloride.

A suitable temperature range for the reaction is from about -20°C to about 125°C, preferably 35°C to 90°C, and more preferably 50°C to 65°C. Pressure is not critical but may depend on the boiling point of the solvent used, i.e., sufficient pressure is needed to maintain a solvent liquid phase. Reaction times are not critical, and can be from several minutes to 48 hours. The reactions are generally carried out in an inert atmosphere, most preferably nitrogen or argon.

The reaction is usually carried out by dissolving the compound (XX¹MLₙL¹ₘ)ₚ, in a suitable solvent, adding the cyclopropene (preferably in a solvent) to a stirred solution of the compound, and optionally heating the mixture until the reaction is complete. The progress of the reaction can be monitored by any of several standard analytical techniques, such as infrared or nuclear magnetic resonance. Isolation of the product can be accomplished by standard procedures, such as evaporating the solvent, washing the solids (e.g., with alcohol or pentane), and then recrystallizing the desired carbene complex. Whether the moieties X, X¹, L, or L¹ are (unidentate) ligands or taken together to form multidentate ligands will depend on the starting compound which simply carries these ligands over into the desired carbene complex.

Under certain circumstances, no solvent is needed.

Reaction temperatures can range from 0°C to 100°C, and are preferably 25°C to 45°C. The ratio of catalyst to olefin is not critical, and can range from 1:5 to 1:30,000, preferably 1:10 to 1:6,000.

Because the carbene compounds mentioned above are stable in the presence of alcohol, thiol, ketone, aldehyde, ester, ether and halogen functional groups, these carbene compounds may be used to catalyze a wide variety of reaction substrates. The added stability also enables one to employ these catalysts in the presence of a protic solvents. This is very unusual among metathesis catalysts and provides a distinct advantage for the process of this invention over the processes of the prior art. Other advantages of the polymerization process of this invention derive from the fact that the carbene compounds are well-defined, stable Ru or Os carbene complexes providing high catalytic activity. Using such compounds as catalysts allows control of the rate of initiation, extent of initiation, and the amount of catalyst.

In one variation of this general procedure, the reaction is conducted in the presence of HgCl₂, preferably 0.01 to 0.2 molar equivalents, more preferably 0.05 to 0.1 equivalents, based on XX¹MLₙL¹ₘ. In this variation, the reaction temperature is preferably 15°C to 65°C.

In a second variation of the general procedure, the reaction is conducted in the presence of ultraviolet radiation. In this variation, the reaction temperature is preferably -20°C to 30°C.

It is also possible to prepare carbene complexes of this invention by ligand exchange. For example, L and/or L¹ can be replaced by a neutral electron donor, L², in compounds of Formula I by reacting L² with compounds of Formula I wherein L, L¹, and L² are independently selected from phosphine, sulfonated phosphine, phosphite, phosphinite, phosphonite, arsine, stibine, ether, amine, amide, sulfoxide, carbonyl, nitrosyl, pyridine or thioether. Similarly, X and/or X¹ can be replaced by an anionic ligand, Y, in compounds of Formula I by reacting M¹Y with compounds of Formula I, wherein Y, X and X¹ are independently selected from halogen, hydrogen, diketonates, or C₁-C₂₀ alkyl, aryl, C₁-C₂₀ alkoxide, aryloxide, C₂-C₂₀ alkoxycarbonyl, arylcarboxylate, C₁-C₂₀ carboxylate, aryl or C₁-C₂₀ alkylsulfonate, C₁-C₂₀ alkylthio, C₁-C₂₀ alkylsulfonyl, C₁-C₂₀ alkylsulfinyl, each optionally substituted with C₁-C₅ alkyl, halogen, C₁-C₅ alkoxy or with a phenyl group optionally substituted with halogen, C₁-C₅ alkyl or C₁-C₅ alkoxy. These ligand exchange reactions are typically carried out in a solvent which is inert under the reaction conditions. Examples of solvents include those described above for the preparation of the carbene complex.

The high level metathesis activity of the carbene compounds also make these compounds useful for catalyzing the ring-closing metathesis of acyclic dienes as described in Fu, G., et al., J. Am. Chem. Soc., 1993, 115:9856-9858 which is incorporated herein by reference.

The carbene compounds may also be used for the preparation of telechelic polymers. Telechelic polymers are macromolecules with one or more reactive end-groups. Telechelic polymers are useful materials for chain extension processes, block copolymer synthesis, reaction injection molding, and network formation. Uses for telechelic polymers and their synthesis is described in Goethals, Telechelic Polymers: Synthesis and Applications (CRC Press: Boca Raton, FL, 1989).

The carbene compounds of the present invention may also be prepared by a one step synthesis as shown in equation below 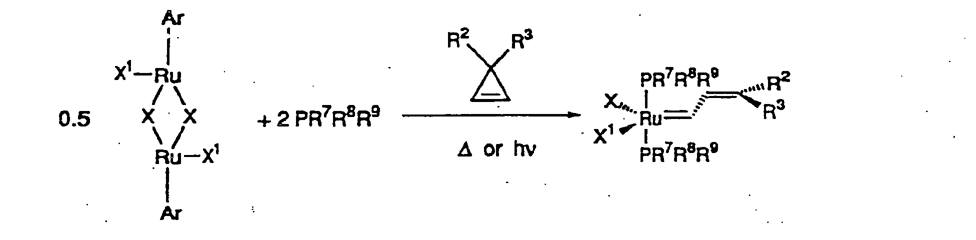 wherein M, X, X¹, R² and R³ are as defined above. Preferably, R² and R³ are independently selected from the group consisting of C₁-C₆ alkyl or phenyl. Ar represents an aromatic compound, preferably a di-, tri-, tetra- or hexa- substituted benzene, most preferably benzene, toluene, xylene, cymene, tetramethylbenzene and hexamethylbenzene. R⁷, R⁸ and R⁹ are independently selected from substituted and unsubstituted C₁-C₁₀ alkyl, secondary alkyl, cycloalkyl and aryl.

Examples of solvents for this reaction include benzene, toluene, xylene and cymene. A suitable temperature range for this reaction is from about 0°C to about 120°C, preferably 45°C to 90°C. The reaction may be conducted in the presence of oxygen. However, it is preferred that it is carried out under an inert atmosphere. The reaction is generally performed under atmospheric pressure. Monitoring the progression of the reaction and isolation of the product can be accomplished by any one of a variety of standard procedures known in the art as described above. Typical reaction conditions for this one step synthesis are provided in Example 1.

The carbene compounds of the present invention may be employed in a variety of olefin metathesis reactions such as those described in U.S. Patent No. 5,312,940 which is incorporated by reference herein.

For most applications, a highly functionalized polymer, i.e., a polymer where the number of functional groups per chain is 2 or greater, is required. Thus, it is desirable that the catalyst used to form the telechelic polymer be stable in the presence of functional groups.

The reaction scheme for a ROMP telechelic polymer synthesis is provided below. In a ROMP telechelic polymer synthesis, acyclic olefins act as chain-transfer agents to regulate the molecular weight of polymers produced. When α,ω-difunctional olefins are employed as chain-transfer agents, difunctional telechelic polymers can be synthesized. As shown in the reaction sequence, the chain-transfer reaction with a symmetric, α,ω-difunctional olefin, the propagating alkylidene is terminated with a functional group, and the new functionally substituted alkylidene reacts with a monomer to initiate a new chain. This process preserves the number of active catalyst centers and leads to symmetric telechelic polymers with a functionality that approaches 2.0. 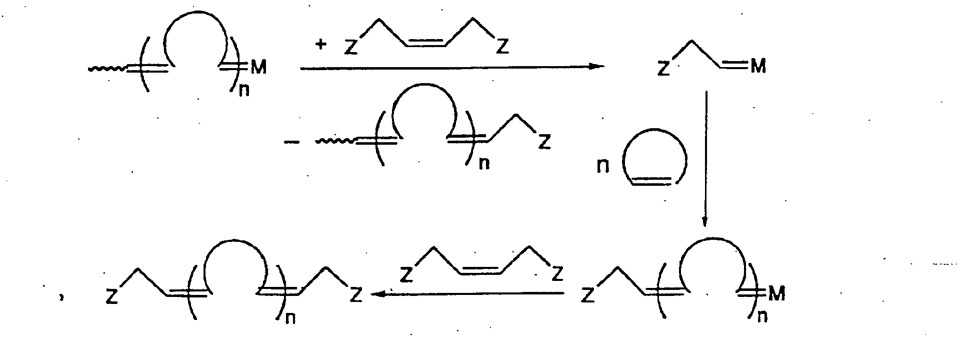 The only polymer end-groups that do not contain residues from the chain-transfer agent are those from the initiating alkylidene and the end-capping reagent. In principle, these end-groups could be chosen to match the end-group from the chain-transfer agent.

Ring opening metathesis polymerization (ROMP) using W(CHAr)(NPh)[OCCH₃(CF₃)₂]₂(THF) has been shown to be a viable polymerization technique for well-defined telechelic polymers. Hillmyer, et al., Macromolecules, 1993, 26:872. However, use of this carbene catalyst for telechelic polymer synthesis is limited by the instability of the tungsten catalyst in the presence of functional groups. The tungsten catalyst is also unstable in the presence of low concentrations of monomers.

The stability of the carbene compounds of the present invention to a wide range of functional groups as well as the ability of these carbene compounds to catalyze ROMP reactions make these compounds particularly desirable for the synthesis of telechelic polymers. The high level metathesis activity of the carbene compounds enable a wider range of cyclic and acyclic olefins to be employed. By way of example, the synthesis of hydroxytelechelic polybutadiene is described in Example 5.

The following examples set forth the synthesis and application of the ruthenium and osmium carbene compounds of the present invention. The following examples also set forth the preferred embodiments of the present invention. Further objectives and advantages of the present invention other than those set forth above will become apparent from the examples which are not intended to limit the scope of the present invention.

The abbreviations Me, Ph, ⁱPr, Cy and THF used in the following examples refer to methyl, phenyl, isopropyl, cyclohexyl and tetrahydrofuran, respectively.

### EXAMPLES

### 1. One Step Synthesis Of Carbene Compounds Of The Invention

The carbene compounds of the present invention may be prepared in a one step synthesis as illustrated in the reaction sequence below.

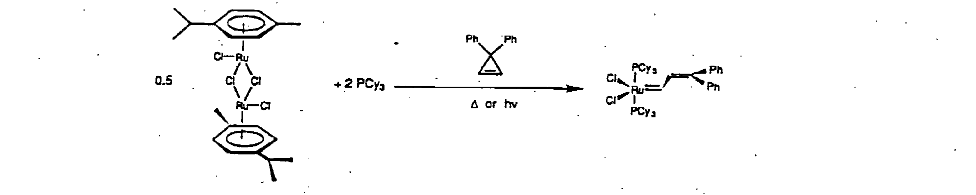

In a typical reaction, [(Cymene)RuCl₂]₂ dimer complex (0.53g, 1.73 mmol Ru) and PCy₃ (0.91 g, 2 equiv) were loaded under inert atmosphere into a 100 mL Sclenk flask equipped with a magnetic stirbar. Benzene (40 mL) was then added followed by 3,3-diphenylcyclopropene (0.33g, 1 equiv). The reaction flask was then attached to a reflux condenser under an inert atmosphere and heated in an oilbath at 83-85°C for 6 hours. The solvent was then removed to complete dryness *in vacuo* and the remaining red solid washed with pentane (4 x 25 mL) under inert atmosphere. The remaining red powder was dried under vacuum for 12 h and stored under an inert atmosphere yielding 1.4 g of Cl₂Ru(PCy₃)₂(=CCH=CPh₂) in 88% yield.

### 2. Effect Of Secondary Alkyl Substituents On Catalyst Turnover Rate

The activity of the carbene catalysts of the present invention has been found to be proportional to the number of secondary alkyl or cycloalkyl substituents on the phosphine. For example, in the reaction

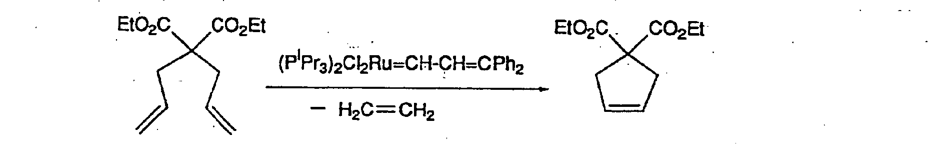

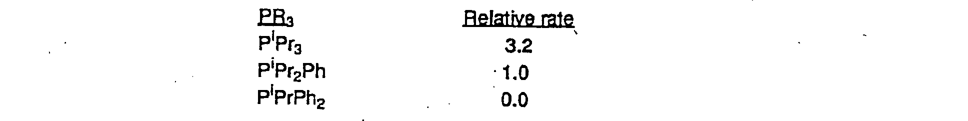

the turnover rate per hour of the catalyst increases as the number of isopropyl substituents on the phosphine increases.

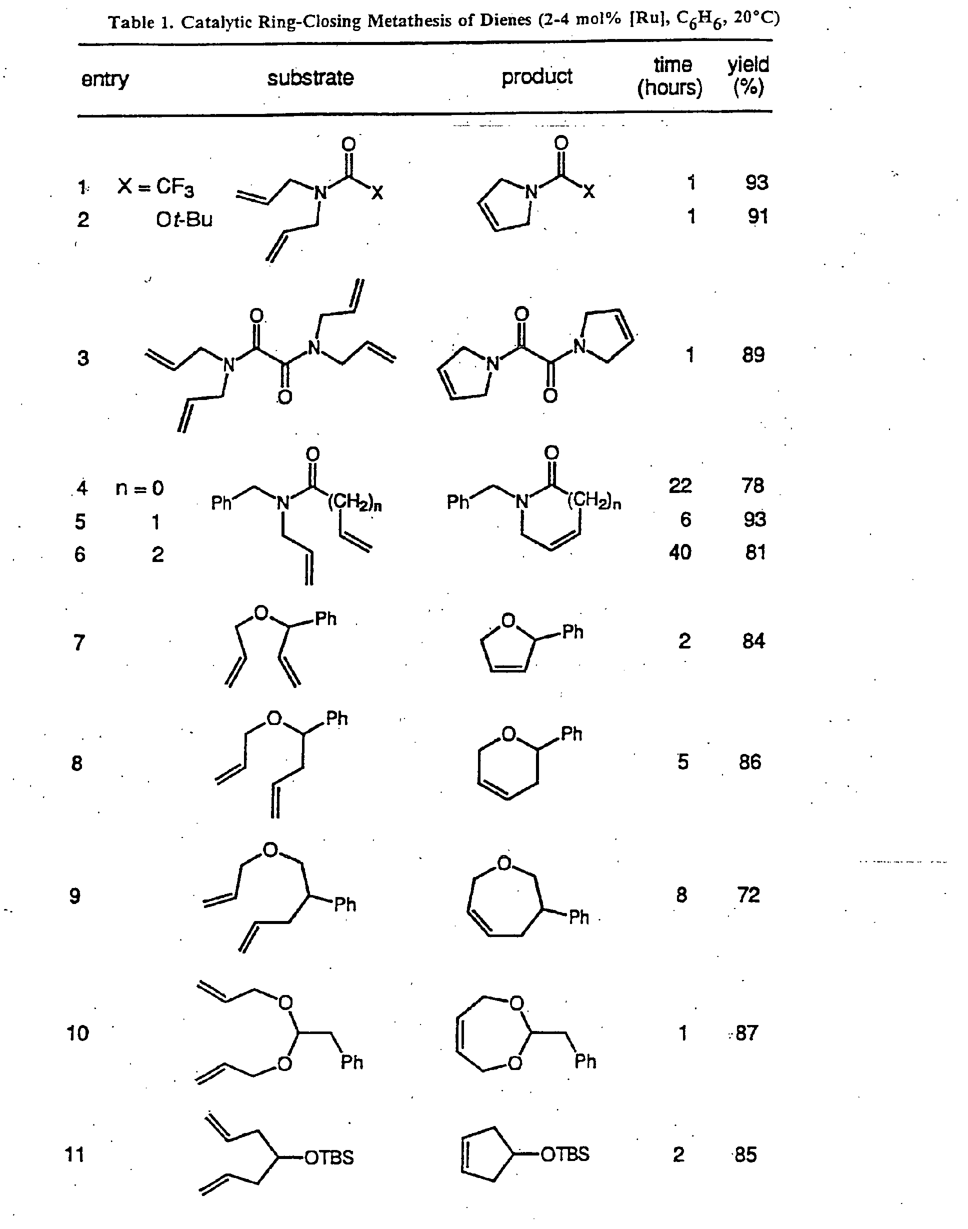

### 3. Ring-Closing Metathesis Of Functionalized Dienes

Table 1 depicts the synthesis of several cycloalkenes from functionalized dienes using Cl₂Ru(PCy₃)₂(=CCH=CPh₂) wherein Cy is cyclohexyl. A typical experimental protocol for performing ring-closing metathesis on the diene of entry 8 of Table 1 is as follows.

The diene of entry 8 (0.50 mmol) was added to a homogeneous orange-red solution of 0.01 mmol Cl₂Ru(PCy₃)₂(=CCH=CPh₂) in 15 mL of dry benzene under argon. The resulting mixture was then stirred at 20°C for 5 h, at which time thin layer chromatography showed the reaction to be complete. The reaction was then quenched by exposure to air (until greenish-black, 6 h), concentrated and purified by flash chromatography (0 -> 6% Et₂O / hexane) to yield dihydropyran as a colorless oil in 86% yield.

### 4. Carbene Catalyzed Polymerization Of 5-Acetoxy-cyclooctene

The carbene compounds of the present invention may be used in the polymerization of nonstrained cyclic olefins such as cyclooctene as depicted in the reaction sequence below. 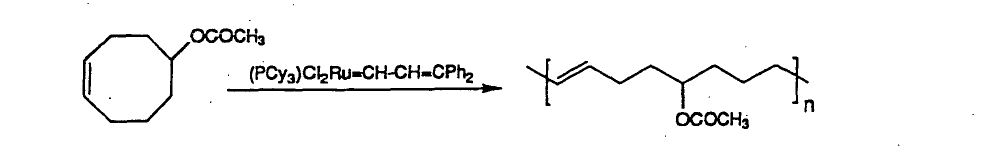 In order to polymerize 5-acetoxy-cyclooctene, a small vial was charged with 2.6 g of degassed 5-acetoxy-cyclooctene and a stirbar. A solution of 15 mg of Cl₂Ru(PCy₃)₂(=CCH=CPh₂) in 200 µL of CH₂Cl₂ was added to the vial under inert atmosphere. The vial was capped and placed in an oil bath at about 48°C. After about 2.5 hours, the red-orange solution became noticeably viscous. After about 5.5 hours, the contents of the vial were solid. After 24 hours, the vial was removed from the oil bath and cooled to room temperature. The cap was removed from the vial and 100 µL of ethyl vinylether, 10 mL of chloroform and about 10 mg of 2,6-di-*tert*-butyl-4-methylphenol (butylated hydroxytoluene) were added to the vial to dissolve the solid, yielding a yellow-orange solution. After about 12 hours of stirring, an additional 20 mL of chloroform was added to the solution. The resulting solution was then poured into about 200 mL of methanol yielding an off-white precipitate. The off-white solid was stirred in the methanol until it appeared free of color. The resulting white solid was then isolated and dried under vacuum in 85% yield (2.2 g).

### 5. Synthesis of Hydroxytelechelic Polybutadiene.

The carbene compounds may also be used to synthesize telechlic polymers such as hydroxytelechelic polybutadiene as described below. A one-neck, 500 mL, Schlenk flask, equipped with a magnetic stirbar, was charged with 1,5-cyclooctadiene (103.3 g, 955 mmol, 3673 equiv). Toluene (103.1 g) and 1,4-diacetoxy-cis-2-butene (11.4 g, 66.2 mmol, 255 equiv) were added to the reaction flask. A stopcock was placed in the neck of the flask and the reaction mixture was stirred, cooled to 0° C, and subjected to vacuum (∼0.05 mm Hg) at 0° C for 30 minutes. The reaction mixture was back-filled with argon, and with a continuous argon flow, Cl₂Ru(PCy₃)₂(CHCHCPh₂) (0.245 g, 0.26 mmol, 1.0 equiv) was added as a solid to the reaction flask while stirring. The stopcock was replaced by a septum, and the system was subjected to vacuum (∼0.05 mm Hg) at 0° C for 10 minutes. The dark red-orange reaction mixture was placed in an oil bath at 45-50° C and stirred for 44 h under a slow purge of argon. The light orange reaction mixture was allowed to warm to room temperature. Vinyl acetate (14 g, 15 mL, 163 mmol, 627 equiv) and BHT (2,6-di-*tert*-butyl-4-methylphenol) (15 mg) were added to the reaction mixture under argon. The mixture was stirred at room temperature for 0.5 h, placed in an oil bath at 45-50° C, and stirred for 7 h. The reaction mixture was allowed to cool to room temperature and poured into 800 mL of methanol. The mixture was stirred overnight and the polymer was isolated by centrifugation. The polymer was then redissolved in 400 mL tetrahydrofuran, cooled to 0° C and 100 mL of 0.7 M sodium methoxide in methanol (70 mmol sodium methoxide) was added at 0° C. The mixture was allowed to stir at 0° C for 3.5 h. Methanol (400 mL) was then added to the reaction mixture to precipitate the polymer. The reaction mixture was allowed to warm to room temperature, stirred overnight, and isolated by centrifugation.

### 6. Metathesis Of Methyl Oleate

In a nitrogen-filled glove box, methyl oleate (3.2g, 2000 equiv) was added to a vial containing a solution of Cl₂(PCy₃)₂Ru=CH-CH=CPh₂ (5 mg in 0.1 mL CH₂Cl₂). The vial was then capped and stirred at room temperature for 4 days. As illustrated in the reaction sequence below, an equilibrium mixture of metathesis products was produced.

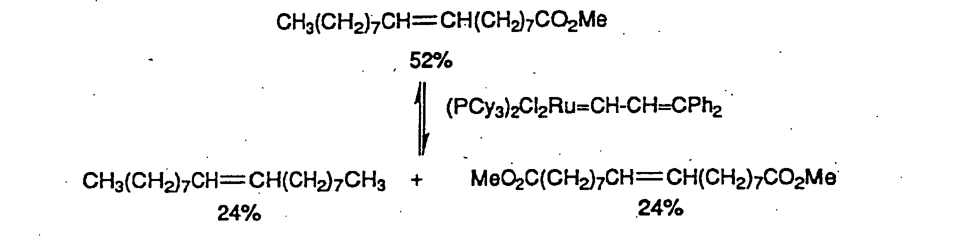

### 7. Metathesis Of Oleic Acid

In a nitrogen-filled glove box, oleic acid (0.3g, 200 equiv) was added to a vial containing a solution of Cl₂(PCy₃)₂Ru=CH-CH=CPh₂ (5 mg in 0.1 mL CH₂Cl₂). The vial was then capped and stirred at room temperature for 4 days. As illustrated in the reaction sequence below, an equilibrium mixture of metathesis products was produced.

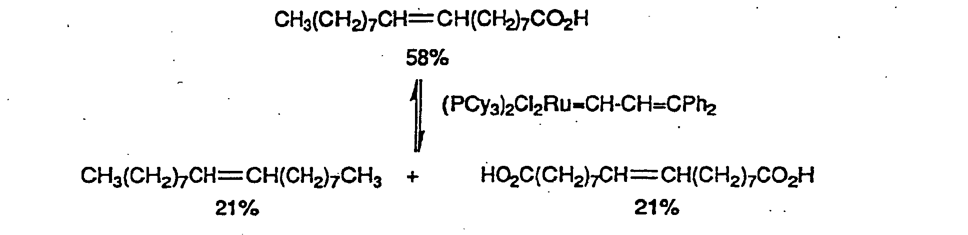

### 8. Metathesis of Methyl Oleate and Ethylene

In a nitrogen-filled glove box, methyl oleate (1 g, 152 equiv) was added to a Fisher-Porter tube containing a solution of Cl₂(PCy₃)₂Ru=CH-CH=CPh₂ (20 mg in 30 mL CH₂Cl₂). The tube was sealed, pressurized to 100 psi of ethylene, and then let stirred at room temperature for 12 hours. As illustrated in the reaction sequence below, an equilibrium mixture of metathesis products was produced.

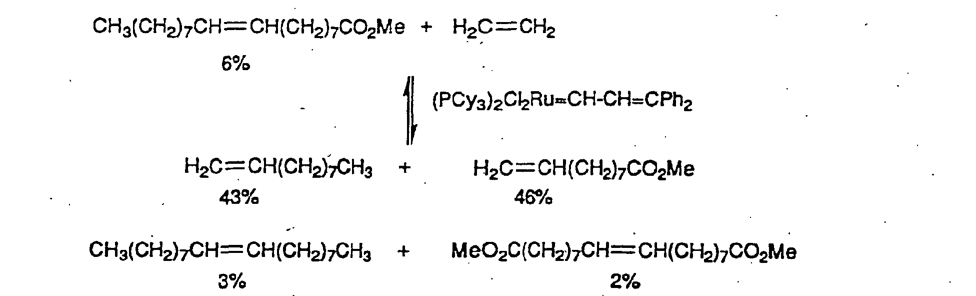

### 9. Metathesis of Oleic Acid and Ethylene

In a nitrogen-filled glove box, oleate acid (0.91 g, 300 equiv) was added to a Fisher-Porter tube containing a solution of Cl₂(PCy₃)₂Ru=CH-CH=CPh₂ (10 mg in 150 mL CH₂Cl₂). The tube was sealed, pressurized to 100 psi of ethylene, and then let stirred at room temperature for 12 hours. As illustrated in the reaction sequence below, an equilibrium mixture of metathesis products was produced.

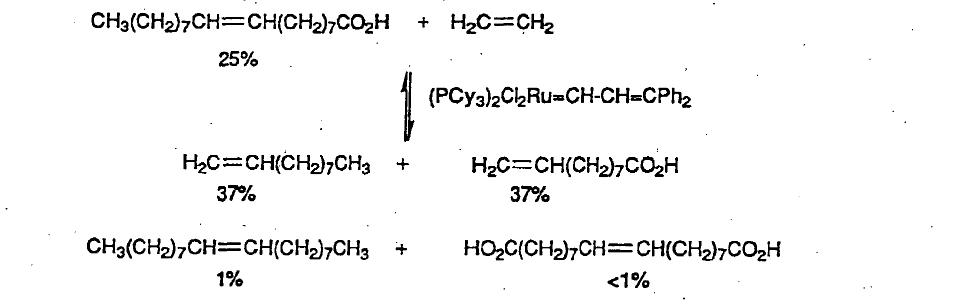

### 10. Depolymerization Of An Unsaturated Polymer With Ethylene

In a nitrogen-filled glove box, polyheptene (0.3 g) was added to a Fisher-Porter tube containing a solution of Cl₂(PCy₃)₂Ru=CH-CH=CPh₂ (20 mg in 5 mL CH₂Cl₂). The tube was sealed, pressurized to 60 psi of ethylene, and then let stirred at room temperature for 24 hours. As illustrated in the reaction sequence below, an equilibrium mixture of 1,8-nonadiene and its ADMET oligomers was produced.

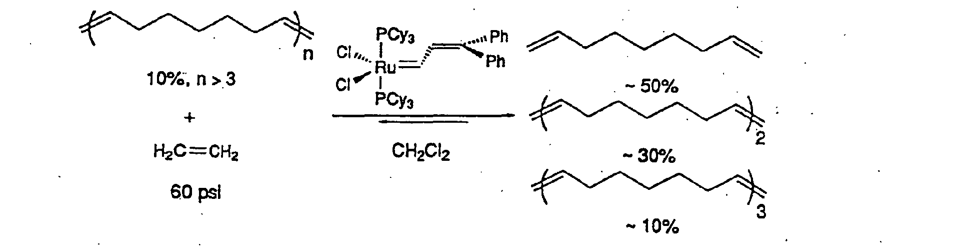

### 11. Synthesis of 1,6-Heptadiene From Cyclopentene

In a nitrogen-filled glove box, cyclopentene (1 g, 680 equiv) was added to a Fisher-Porter tube containing a solution of Cl₂(PCy₃)₂Ru=CH-CH=CPh₂ (20 mg in 5 mL CCl₄). The tube was sealed, pressurized to 60 psi of ethylene, and then let stirred at room temperature for 24 h. As illustrated in the reaction sequence below, an equilibrium mixture of 1,7-heptadiene and its ADMET oligomers was produced.

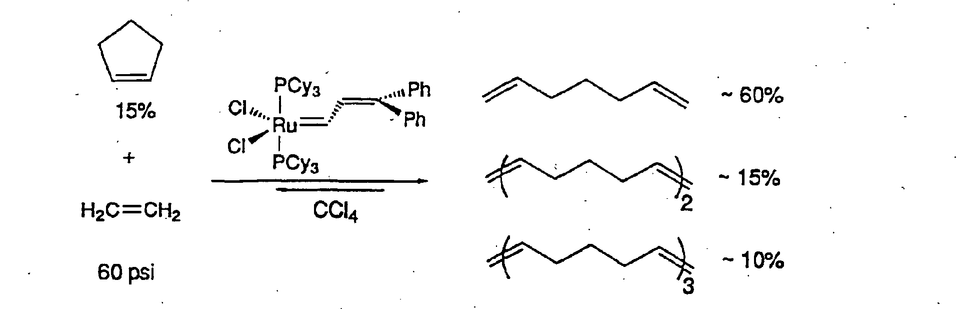

### 12. Ruthenium Carbene Catalyzed Polymerization of Dicyclopentadiene

A small Schlenk flask equipped with a small magnetic stir bar was charged with about 9.7 g of dicyclopentadiene (DCP) (Aldrich, 95%, inhibited with 200 ppm *p-tert*-butylcatechol (catalog # 11,279-8)). The flask was stoppered with a greased ground glass stopper and placed in an oil bath at about 38°C. The DCP flask was subjected to vacuum (a reduced pressure of about 0.05 mmHg) and stirred for 30 minutes. Next, the flask was cooled to about 0°C in an ice water bath for 5 minutes after which the DCP was solid. The flask was then back-filled with argon, the -stopper was removed, and (PCy₃)₂Cl₂Ru=CH-CH=CPh₂(20 mg) was added as a solid (no special precautions were taken to avoid atmospheric oxygen). The stopper was replaced, and the solids were subjected to vacuum for 10 minutes at about 0°C. The flask was placed in an oil bath at about 38°C for 5 minutes while keeping its contents under vacuum. During this time the DCP liquefied, and the catalyst dissolved in the DCP to-yield a non-viscous, red solution which appeared homogeneous. The stir bar was removed from the bottom of the flask with the aid of another magnet, and the temperature was raised to about 65°C while keeping the contents of the flask under vacuum. When the temperature of the oil bath reached about 55°C (about 2 minutes after the heating was initiated), the contents of the flask became yellow-orange and appeared to be solid: The temperature of the oil bath was maintained at about 65°C for 1 hour. The flask was removed from the oil bath, back filled with air, broken, and the solid plug of polymer was removed. The polymer was washed with pentane and placed in an oven at about 130°C for 3 hours. The polymer was removed from the oven, cooled to room temperature, and weighed (8.3 g; 86%, [DCP]/[Ru] ∼ 2900). (Losses due the removal of volatiles during the degassing were not taken into account in the calculation of the yield.)

### 13. Method of Preparing Compounds Of This Invention From Cyclopropene

A 50 mL Schlenk flask equipped with a magnetic stirbar is charged with (MXX¹LₙL¹ₘ)p (0.1 mmol) inside a nitrogen-filled drybox. Methylene chloride (2 mL) is added to dissolve the complex followed by 25 mL of benzene to dilute the solution. One equivalent of a cyclopropene is then added to the solution. The reaction flask is then capped with a stopper, removed from the box, attached to a reflux condenser under argon and heated at 55°C. The reaction is then monitored by NMR spectroscopy until all the reactants have been converted to the product. At the end of the reaction, the solution is allowed to cool to room temperature under argon and filtered into another Schlenk flask via a cannula filter. The solvent is then removed *in vacuo* to give a solid. This solid is then washed with a solvent in which the by-product of the reaction is soluble but the desired product is not. After the washing the product, the supernatant is removed and the resulting solid powder is dried *in vacuo* overnight. Further purification via crystallization can be performed if necessary.

Representative compounds of the present invention which may be prepared in accordance with the procedure described above are exemplified in Table 2.

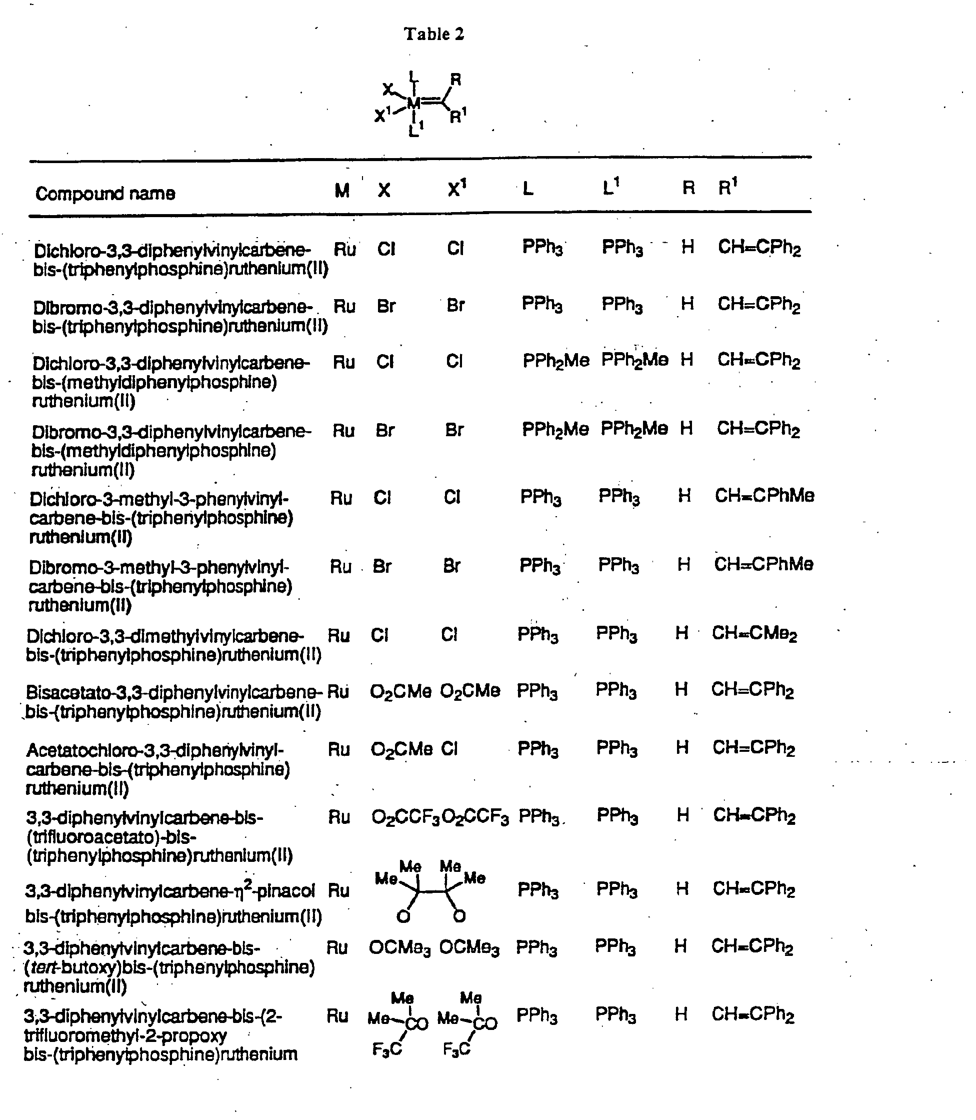

These are representative examples of the ruthenium complexes. Analogous complexes can be made with osmium.

### 14. Synthesis of:

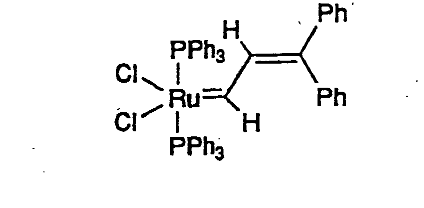

In a typical reaction, a 200 mL Schlenk flask equipped with a magnetic stirbar was charged with RuCl₂(PPh₃)₄ (6.00 g, 4.91 mmol) inside a nitrogen-filled drybox. Methylene chloride (40 mL) was added to dissolve the complex followed by 100 mL of benzene to dilute the solution. 3,3-Diphenylcyclopropene (954 mg, 1.01 equiv) was then added to the solution via pipette. The reaction flask was capped with a stopper, removed from the box, attached to a reflux condenser under argon and heated at 53°C for 11 h. After allowing the solution to cool to room temperature, all the solvent was removed *in vacuo* to give a dark yellow-brown solid. Benzene (10 mL) was added to the solid and subsequent swirling of the mixture broke the solid into a fine powder. Pentane (80 mL) was then slowly added to the mixture via cannula while stirring vigorously. The mixture was stirred at room temperature for 1 h and allowed to settle before the supernatant was removed via cannula filtration. This washing procedure was repeated twice more to ensure the complete removal of all phosphine by-products. The resulting solid was then dried under vacuum overnight to afford 4.28 g (98%) of Compound 1 as a yellow powder with a slight green tint. ¹ 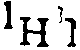 NMR (C₆D₆):δ 17.94 (pseudo-quartet = two overlapping triplets, 1H, Ru=CH, J_{HH}=10.2 Hz, J_{PH}=9.7 Hz), 8.70 (d, 1H, CH=CPh₂, J_{HH} 10.2 Hz). ³¹P NMR (C₆D₆): δ 28.2 (s). ¹³C NMR (CD₂Cl₂): δ 288.9 (t, M = C, J_{CP}=10.4 Hz) 149.9 (t, CH=CPh_{2"} J_{CP}=11.58 Hz).

. The carbene complex which is the compound formed in the above example is stable in the presence of water, alcohol, acetic acid, HCl in ether and benzaldehyde.

### 15. Synthesis of:

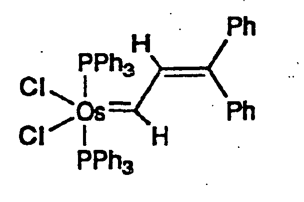

A 50 mL Schlenk flask equipped with a magnetic stirbar was charged with OsCl₂(PPh₃)₃ (100 mg, 0.095 mmol) inside a nitrogen-filled drybox. Methylene chloride (2 mL) was added to dissolve the complex followed by 25 mL of benzene to dilute the solution. 3,3-diphenylcyclopropene (18.53 mg, 1.01 equiv) was then added to the solution via pipet. The reaction flask was capped with a stopper, removed from the box, attached to a reflux condenser under argon and heated at 55°C for 14 h. After allowing the solution to cool to room temperature, all the solvent was removed *in vacuo* to give a dark yellow-brown solid. Benzene (2 mL) was added to the solid and subsequent swirling of the mixture broke the solid into a fine powder. Pentane (30 mL) was then slowly added to the mixture via cannula while stirring vigorously. The mixture was stirred at room temperature for 1 h and allowed to settle before the supernatant was removed via cannula filtration. This washing procedure was repeated two more times to ensure the complete removal of all phosphine by-products. The resulting solid was then dried under vacuum overnight to afford 74.7 mg of Cl₂(PPh₃)₂Os(=CHCH=CPh₂) as a yellow powder (80%). ¹H NMR (C₆D₆): δ 19.89 (pseudo-quartet = two overlapping triplets, 1H, Os = CH, J_{HH} = 10.2 Hz), 8.23 (d, 1H, CH=CPh₂, J_{HH} = 10.2 Hz). ³¹P NMR (C₆D₆): δ 4.98 (s).

### 16. Synthesis of:

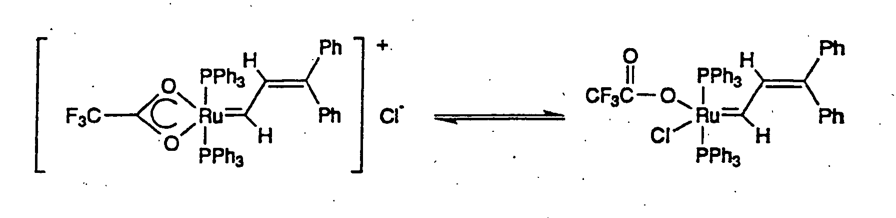

A 50 mL Schlenk flask equipped with a magnetic stirbar was charged with
RuCl₂(PPh₃)₂(=CHCH=CPh₂) (100 mg, 0.18 mmol) inside a nitrogen-filled drybox. Methylene chloride (10 mL) was added to dissolve the complex. AgCF₃CO₂ (24.9 mg., 1 equiv) was weighed into a 10 ml round-bottom flask, dissolved with 3 ml of THF. Both flasks were then capped with rubber septa and removed from the box. The Schlenk flask was. then put under an argon atmosphere and the AgCF₃CO₂ solution was added dropwise to this solution via a gas-tight syringe over a period of 5 min while stirring. At the end of the addition, there was a lot of precipitate in the reaction mixture and the solution turned a fluorescent green color. The supernatant was transferred into another 50 mL Schlenk flask under argon atmosphere via the use of a cannula filter. Subsequent solvent removal *in vacuo* and washing with pentane (10 mL) afforded a green solid powder of the above depicted compound. Yield = 92.4 mg (85%). ¹H NMR (2:2:1 CD₂Cl₂:C₆D₆:THF-d₈): δ 18.77 (dt, 1H, Ru=CH, J_{HH}=11.2 Hz, J_{PH}=8.6 Hz), 8.40 (d, 1H), CH=CPh₂, J_{HH}=11.2 Hz). ³¹P NMR (2:2:1 CD₂Cl₂:C₆D₆:THF-d₈):δ 29.4. ¹⁹F NMR (2:2:1 CD₂Cl₂:C₆D₆:THF-d₈):δ 75.8.

### 17. Synthesis of:

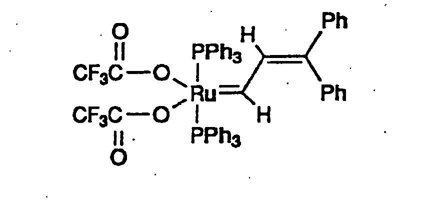

A 50 mL Schlenk flask equipped with a magnetic stirbar was charged with RuCl₂(PPh₃)₂(=CH-CH=CPh₂) (100 mg, 0.11 mmol) inside a nitrogen-filled drybox. Methylene chloride (10 mL) was added to dissolve the complex. AgCF₃CO₂ (49.8 mg, 2 equiv) was weighed into a 10 mL round-bottom flask, dissolved with 4 mL of THF. Both flasks were then capped with rubber septa and removed from the box. The Schlenk flask was then put under an argon atmosphere and the AgCF₃CO₂ solution was added dropwise via a gas tight syringe over a period of 5 min to the solution of ruthenium compound while stirring. At the end of the addition, there was a lot of precipitate in the reaction mixture and the solution turned into a fluorescent lime green color. The supernatant was transferred into another 50 mL Schlenk flask under argon atmosphere with the use of a cannula filter. Subsequent solvent removal in vacuo and washing with pentane (10 mL) afforded the above depicted compound as a green powder. Yield = 102 mg (87%) ¹H NMR (2:2:1 CD₂Cl₂:C₆D₆:THF-d₈) δ 19.23 (dt, slightly overlapping) Ru=CH, J_{HH}=11.5 Hz, J_{PH}=5.4 Hz), 8.07 (d, 1H, CH=CPH2, J_{HH}=11.5 Hz). ³¹P NMR (2:2:1 CD₂Cl₂:C₆D₆:THF-d₈):δ 28.6. ¹⁹F NMR (2:2:1 CD₂Cl₂:C₆D₆:THF-d₈):δ -75.7.

### 18. Synthesis of:

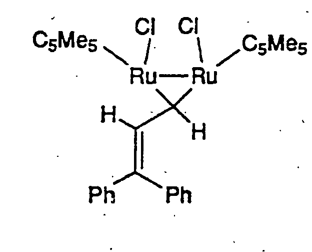

The reaction between [Ru(C₅Me₅)Cl]₄ and 3,3-diphenylcyclopropene was done under a nitrogen atmosphere. [Ru(C₅Me₅)Cl]₄ (100 mg, 0.092 mmoL) was dissolved in 10 mL of tetrahydrofuran. To this solution was added 3,3-diphenylcyclopropene (350 mg, 1.82 mmol). The resulting solution was stirred at room temperature for 1 h. Petroleum ether (10 mL) was then added to the reaction mixture. The reaction was stirred for an additional 30 min, after which all volatile components were removed from the reaction mixture under vacuum. The crude product was extracted with diethyl ether; volatiles were removed from the filtrate under vacuum to afford a dark colored, oily solid. The crude product was further extracted with petroleum ether; volatiles were removed from the filtrate under vacuum to afford a very dark red-brown oil. Recrystalization from petroleum ether at -40°C afforded dark crystals. The NMR spectra of the product was consistent with the formulation [(C₅Me₅)RuCl]₂ (=CH-CH=CPh₂).

### 19. Polymerization of Norbornene Using Compound of Example 14

(PPh₃)₂Cl₂Ru=CH-CH=CPh₂ catalyzed the polymerization of norbornene in a 1:8 mixture of CH₂Cl₂/C₆H₆ at room temperature to yield polynorbornene. A new signal, attributed to H_{α} of the propagating carbene, was observed by ¹H NMR spectroscopy at 17.79 ppm. Its identity and stability was confirmed by preparing a block polymer with 2,3-dideuteronorbornene and perprotionorbornene. When 2,3-dideuteronorbomene was added to the propagating species, the new carbene signal vanished and then reappeared when perprotionorbornene was added for the third block.

### 20. Polymerization of Norbornene Using Compound of Example 18

[(C₅Me₅)RuCl]₂(=CH-CH-CPh₂) (14 mg, 0.030 mmol) was dissolved in 1 mL of perdeuterated toluene under a nitrogen atmosphere. To this was added norbomene (109 mg, 1.16 mmol). The reaction mixture became viscous within minutes as the norbomene polymerized. After 20 h at room temperature a ¹H NMR spectrum of the reaction mixture was taken, which showed polynorbornene and unreacted norbornene monomer in a ratio of 82:12.

### 21. Synthesis of:

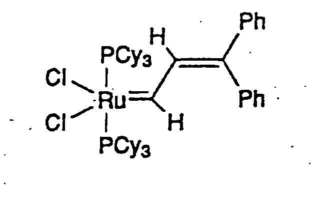

In a typical reaction, a 100 mL Schlenk flask equipped with a magnetic stirbar was charged with (Ph₃P)₂Cl₂Ru=CH-CH=CPh₂ (3.624 g, 4.08 mmol) and PCy₃ (2.4 g, 2.1 equiv) inside a nitrogen-filled drybox. Methylene chloride (60 mL) was added to dissolve the mixture. The reaction flask was capped with a stopper, removed from the drybox, and stirred under argon overnight during which time the reaction mixture turned red. The reaction mixture was then cannula-filtered under argon into another Schlenk flask. The red filtrate was then concentrated under *in vacuo* to about 15 mL. Pentane (60 mL) was slowly added to the mixture via cannula while stirring vigorously. A flocculent green solid, consisting mostly starting material, begins to be separated out of the solution when about 40 mL of pentane is added. The red supernatant was quickly transferred into another Schlenk flask via cannula filtration and then evaporated to dryness under *in vacuo*. The remaining red solid was washed with pentane (3 x 40 mL). To ensure the complete removal of all phosphine by-products, each wash was stirred at room temperature for at least 30 minutes before the supernatant was cannula-filtered away. The resulting solid was then dried under vacuum overnight to afford 3.39 g (ca. 90%) of (Cy₃P)₂Cl₂Ru=CH-CH=CPh₂ as a red powder.

While the present invention is disclosed by reference to the preferred embodiments and examples detailed above, it is to be understood that these examples are intended in an illustrative rather than limiting sense, and it is contemplated that modifications within the spirit and scope of the invention will readily occur to those skilled in the art, which modifications are intended to be encompassed within the scope of the appended claims.

**Divisional 1**

| **Claim Number** | **Support** |
|---|---|
| 1 | Example 12; page 1 lines 26-35 |
| 2 | Page 8 line 17-21;page 11 line 17-25 |
| 3 | Example 12 |
| 4 | Example 12 |
| 5 | Page 2 line 16-19; page 6 line 46-48 and 19-21; page 10 line 34-page 11 line 1 |
| 6 | Example 12 |
| 7 | Original claim 1 |
| 8 | Page 9 line 33-38 |
| 9 | Original claims 1 and 6 |
| 10 | Page 1 lines 37-38 |
| 11 | Page 1 line 29-40 |
| 12 | Page 2 lines 1-2 |
| 13 | Original claim 4; page 6 lines 9-10; page 2 lines 2-3 |
| 14 | Original claim 5 |
| 15 | Original claim 13 |
| 16 | Page 7 line 1-2 |
| 17 | Example 12 |
| 18 | Preceding claims |

## Claims

1. A method for olefin metathesis polymerization, comprising
contacting an olefin with a compound of the formula 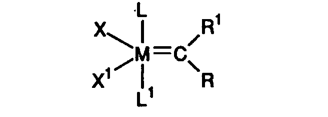 wherein:
the olefin is dicyclopentadiene;
M is Os or Ru;
R and R¹ are independently selected from hydrogen or a hydrocarbon selected from the group consisting of C₂-C₂₀ alkenyl, C₂-C₂₀ alkynyl, C₁-C₂₀ alkyl, aryl, C₁-C₂₀ carboxylate, C₂-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl, and C₁-C₂₀ alkylsulfinyl;
X and X¹ are independently selected from any anionic ligand; and
L is any neutral electron donor; and
L¹ is a trialkylphosphine ligand where at least one of the alkyl groups is a secondary alkyl or a cycloalkyl.

2. The method as in claim 1 wherein X and X¹ are independently selected from halogen, hydrogen, substituted moiety, and unsubstituted moiety wherein the moiety is selected from the group consisting of diketonates, C₁-C₂₀ alkyl, aryl, C₁-C₂₀ alkoxide, aryloxide, C₂-C₂₀ alkoxycarbonyl, arylcarboxylate, C₁-C₂₀ carboxylate, arylsulfonate, C₁-C₂₀ alkylsulfonate, C₁-C₂₀ alkylthio, C₁-C₂₀ alkylsulfonyl, and C₁-C₂₀ alkylsulfinyl, wherein the moiety substitution is selected from the group consisting of C₁-C₅ alkyl, halogen, C₁-C₅ alkoxy, unsubstituted phenyl, halogen substituted phenyl, C₁-C₅ alkyl substituted phenyl, and C₁-C₅ alkoxy substituted phenyl.

3. The method as in any one of claim 1 or claim 2 wherein the dicyclopentadiene has a purity of 95% or less.

4. The method as in claim 3 wherein the dicyclopentadiene has a purity of about 95%.

5. The method as in any one of claims 1 to 4 wherein the dicyclopentadiene includes a functional group selected from the group consisting of alcohol, thiol, ketone, aldehyde, ester, ether, amine, amide, nitro acid, carboxylic acid, disulfide, carbonate, carboalkoxy, and halogen.

6. The method as in any one of claims 1 to 4 wherein the dicyclopentadiene is unsubstituted dicyclopentadiene.

7. The method as in any one of the preceding claims wherein L and L¹ each are of the formula PR³R⁴R⁵, wherein R³ is a secondary alkyl or a cycloalkyl, and R⁴ and R⁵ are each independently selected from the group consisting of C₁-C₁₀ primary alkyl, secondary alkyl, and cycloalkyl.

8. The method as in claim 7 wherein the catalyst is one of 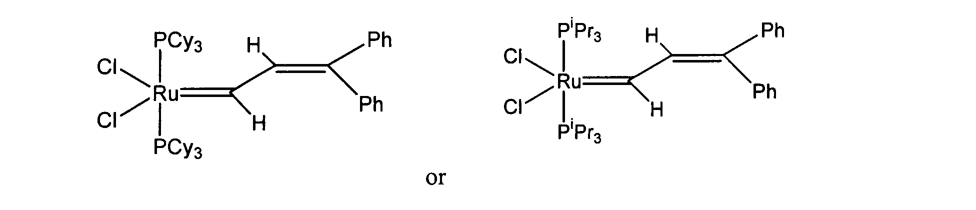 wherein ⁱPr is isopropyl and Cy is cyclohexyl.

9. A method for olefin metathesis polymerization, comprising:
contacting an olefin with a compound of the formula: 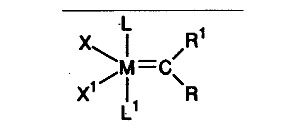 wherein:
the olefin is dicyclopentadiene;
M is Os or Ru;
R and R¹ are independently selected from hydrogen or a hydrocarbon selected from the group consisting of C₁-C₂₀ alkyl, C₂-C₂₀ alkenyl, C₂-C₂₀ alkynyl, C₂-C₂₀ alkoxycarbonyl, aryl, C₁-C₂₀ carboxylate, C₁-C₂₀ alkoxy, C₂-C₂₀ alkenyloxy, C₂-C₂₀ alkynyloxy and aryloxy;
X and X¹ are independently selected from any anionic ligand; and
L and L¹ are independently selected from PR³R⁴R⁵ wherein R³ is selected from the group consisting of secondary alkyl and cycloalkyl and wherein R⁴ and R⁵ are independently selected from aryl, C₁-C₁₀ primary alkyl, secondary alkyl, and cycloalkyl.

10. A process as in claim 9 wherein said hydrocarbon is substituted with a member of the group consisting of C₁-C₅ alkyl, halogen, C₁-C₅ alkoxy and phenyl.

11. A process as in claim 10 wherein said phenyl is substituted with a member of the group consisting of halogen, C₁-C₅ alkyl or C₁-C₅ alkoxy.

12. The method as in any one of claims 7 to 11 wherein R³, R⁴ and R⁵ are either the same or different and are secondary alkyl or cycloalkyl.

13. The method as in any one of claims 7 to 11 wherein R³, R⁴, and R⁵ are either the same or different and are isopropyl, isobutyl, sec-butyl, neopentyl, neophenyl, cyclopentyl or cyclohexyl.

14. The method as in claim 13 wherein L and L¹ are either the same or different and are P(isopropyl)₃, P(cyclopentyl)₃ or P(cyclohexyl)₃.

15. The method as in any one of the preceding claims wherein the polymerization occurs in the absence of solvent.

16. The method as in any one of claims 1 to 14 wherein the process is conducted in a protic solvent, aqueous solvent, organic solvent or mixtures thereof.

17. A method for olefin metathesis polymerization, comprising:
contacting an olefin with a compound of the formula: 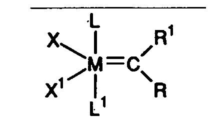 wherein:
the olefin is dicyclopentadiene;
M is Ru;
R is hydrogen;
R¹ is CHCPh₂;
X and X¹ are both Cl; and
L and L¹ are both PCy₃.

18. Use of a compound having the formula 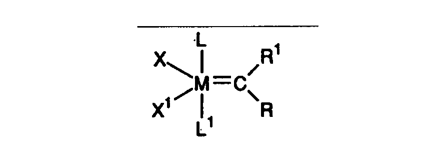 as a catalyst for the polymerization of dicyclopentadiene, wherein M, R, R¹, X, X¹, L and L¹ are as defined in any one of the preceding claims.
